# EUROPEAN PATENT SPECIFICATION

(11) **EP 3 061 730 B1**
(45) Date of publication and mention of the grant of the patent: **10.04.2019**
(21) Application number: 16157147.6
(22) Date of filing: 24.02.2016
(51) Int. Cl.: C02F 1/00, C02F 11/04, G01N 21/90, G01N 35/10

(54) **DIGESTER AND METHOD FOR DETECTING A PRESENCE OF FOAM IN A DIGESTER**
FAULBEHÄLTER UND VERFAHREN ZUR ERKENNUNG DER ANWESENHEIT VON SCHAUM IN EINEM FAULBEHÄLTER
DIGESTEUR ET PROCÉDÉ POUR DÉTECTER LA PRÉSENCE DE MOUSSE DANS UN LESSIVEUR

(30) Priority: 24.02.2015 NL 2014342
(43) Date of publication of application: 31.08.2016
(73) Proprietor: Host Holding B.V., 7521 PS Enschede (NL)
(72) Inventor: Te Braak, Marcel Anton Franciscus, 7577 LM Oldenzaal (NL)
(74) Representative: Patentwerk B.V.

(56) References cited:
- EP-A1- 2 453 233
- WO-A1-2007/092986
- DE-A1-102013 000 977
- US-A- 3 739 795
- US-A- 5 463 895
- PEUKERT W: "The Optimisation of Fermentation Processes Utilising Integrated Off-Gas Separation", FILTRATION AND SEPARATION, ELSEVIER ADVANCED TECHNOLOGY, OXFORD, GB, vol. 35, no. 6, 8 July 1998 (1998-07-08), XP004137690, ISSN: 0015-1882, DOI: 10.1016/S0015-1882(98)80015-3

## Description

The invention relates to a digester, comprising a container for containing biomass and/or sludge and/or a digestate thereof, preferably up to an intended level of said container.

It is noted that said biomass may be any type of biomass. Said sludge may be any type of sludge, for example wastewater sludge.

Such a digester is known per se. Said container may be filled with biomass, preferably up to said intended level. The intended level may be chosen as desired. For example, said intended level may be a preferred maximum level, or any other suitable level. During use of said digester, wherein said container contains said biomass and/or sludge and/or digestate thereof, and if no biomass and/or sludge is fed or discharged from said container, the level of biomass and/or sludge and/or digestate in the container is substantially constant, and preferably said intended level.

Above the level of biomass and/or sludge and/or digestate thereof, it is possible that a foam is formed. Such foam is undesired. It is therefor an object of the invention to provide a digester according to the preamble, which is able to detect any foam present above the biomass and/or sludge and/or digestate thereof. If any foam is detected, for example an anti foaming agent may be fed to said container for reducing or removing the foam. The document *"*The Optimisation of Fermentation Processes Utilising Integrated Off-Gas Separation", Filtration & Separation, July/August 1998, pp.499-503, discloses a foam detecting apparatus known from the prior art.

The above stated object is achieved by:
- a first pressure sensor for measuring a first pressure of gas present in said container, said first pressure sensor being in medium through flow connection with a first, upper part of said container, and
- a second pressure sensor for measuring a second pressure of gas and/or foam present in said container, said second pressure sensor being in medium through flow connection with a second part of said container above said intended level and below said first, upper part, wherein the digester comprises a tube connecting said second pressure sensor with said second part of the container, said tube having an inlet opening arranged just above said intended level.

If no foam is present in said container above the level of biomass and/or sludge and/or digestate thereof, both the first and second pressure sensor will measure substantially the same pressure. If foam is present in said container above the level of biomass and/or sludge and/or digestate thereof, said second pressure will indicate a higher pressure than said first pressure. A difference between the second pressure and the first pressure is therefor an indication of foam being present in said container. With use of such two pressure sensors, the presence of foam in the container may thus easily and/or accurately be detected.

An advantage of using two pressure sensors for measuring the first and second pressure is that the absolute pressure in each of the first and second part of the container is provided.

The digester according to the invention may in particular be a separator digester. In such a digester based on separation biomass and/or sludge and/or a digestate thereof separates under the influence of gravity into stratified layers based on the specific density of the particles and/or substances present in the biomass and/or sludge and/or digestate thereof. The digester is in particular not a bubble column. The pressures measured by the first and second pressure sensors may be read by a person, who may thereby obtain information on the presence of foam in the container.

Alternatively, said digester may comprise a means arranged to compare said first and second pressures, wherein a difference in said first and second pressures is an indication of foam present in said container above said intended level. Such a means may automatically detect foam present in said container.

In an embodiment of the digester according to the invention said first and second pressure sensor are integrally formed as one differential pressure sensor, said differential pressure sensor being on the one hand in said medium through flow connection with said first, upper part of said container and on the other hand in said medium through flow connection with said second part of said container, said differential pressure sensor being arranged to provide a difference in pressure between said first and second parts of said container as an indication of foam present in said container above said intended level.

This embodiment may provide a simple digester in which absolute pressures in the first and second part of the container may not be obtained by said differential pressure sensor, but the difference in pressure between the first and second part may be provided, thereby indicating whether foam is present in said container above said intended level.

The invention may thus also relate to a digester, comprising a container for containing biomass and/or sludge and/or a digestate thereof, up to an intended level of said container, characterized by:
- a differential pressure sensor, said differential pressure sensor being on the one hand in a medium through flow connection with a first, upper part of said container and on the other hand in a medium through flow connection with a second part of said container, preferably just above said intended level, said differential pressure sensor being arranged to provide a difference in pressure between said first and second parts of said container as an indication of foam present in said container above said intended level.

In an embodiment of the digester according to the invention, said container comprises a foam outlet, said foam outlet being arranged in an area of said container that is below said first part of said container and above said second part of said container.

Said foam outlet is arranged to discharge foam from said container if the level of foam increases too much. By providing said foam outlet below said first part of container said first pressure sensor cannot come into contact with the foam, such that a pressure difference between the first part comprising no foam and the second part optionally comprising foam may be obtained and the optional presence of foam may accurately be detected.

In another embodiment of the digester according to the invention, said container comprises a overflow outlet, said overflow outlet being arranged in an area of said container that is below said second part of said container.

By providing such an overflow outlet, said biomass and/or sludge is automatically discharged from said container if the biomass and/or sludge exceeds the level of the area where said overflow outlet is provided. This way it is easily prevented that said biomass and/or sludge reaches the second part of the container where said second pressure is measured.

Said overflow outlet defines a maximum level of biomass and/or sludge and/or digestate thereof that can be present in the container. Said intended level may be arranged below or at the same level as said overflow outlet. If said intended level is as the same level as said overflow outlet said outlet defines the intended level. In that case filling of said container with biomass and/or sludge is preferably stopped as soon as any biomass and/or sludge is discharged by said overflow outlet, indicating that said container is filled up to the intended level.

In yet another embodiment of the digester according to the invention, said digester comprises a hydrostatic pressure sensor arranged to indicate a level of biomass and/or sludge and/or digestate thereof contained in said container.

By measuring a hydrostatic pressure of said biomass and/or sludge and/or digestate thereof contained in the container, the level of biomass and/or sludge and/or digestate contained therein may easily be determined. By determining the level of biomass and/or sludge and/or digestate thereof contained in the container, it is possible to determine if the container is filled up to the intended level. This may for example be performed by comparing the measured hydrostatic pressure or level determined thereby with a determined hydrostatic pressure or level, wherein the container is filled up to the intended level if the measured hydrostatic pressure or level determined thereby substantially equals the determined hydrostatic pressure or level.

Practically said digester comprises a signaling means connected to said hydrostatic pressure sensor for signaling that the container is filled up to the intended level. This way, filling of the container may be stopped as soon as the signaling means make a signal that the container is filled up to the intended level.

In yet another embodiment of the digester according to the invention, said digester comprises an indicating means for indicating the intended level.

Said indicating means may for example comprise a marking arranged in said container.

According to the invention, the digester comprises a tube connecting said second pressure sensor with said second part of said container.

Said tube has an inlet opening arranged just above said intended level. Foam present in said container may enter the tube, thereby increasing the pressure in the tube, wherein the pressure in the tube is measured by said second pressure sensor.

Said tube is preferably arranged substantially vertical in said container.

An advantage thereof is that any foam present in the tube may automatically move downwards in said tube as a result of the gravitational force and leave said tube via the inlet opening if the foam is removed from said container. This way, the tube is automatically, self-cleaned, such that the second pressure sensor may accurately measure the pressure of the gas after removal of the foam. As a result of the self-cleaning effect of the tube the pressure measurements and thereby the foam detection may be performed accurately and reliable even after the presence of any foam. It is noted that said inlet opening also functions as the outlet opening.

Practically said second part of said container is between 5 - 30 cm, preferably between 10 - 20 cm, above said intended level.

The invention further relates to a method for detecting a presence of foam in a digester according to any of the above mentioned embodiments, said method comprising the steps of:
(a) filling said container with biomass and/or sludge, up to said intended level;
(b) measuring said first and second pressures, and
(c) comparing said first and second pressures, wherein a difference in said first and second pressures is an indication of foam present in said container above said intended level.

Steps (b) and/or (c) may be performed repeatedly with any desired time interval, such that the optional presence of foam in the container may be detected repeatedly.

In an embodiment of the method according to the invention steps (b) and (c) are performed by measuring a difference in pressure between said first and second parts of said container using said differential pressure sensor.

In an embodiment of the method according to the invention, the method comprises the further steps of:
(d) determining if said difference obtained in step (c) exceeds a certain threshold; and
(e) providing an anti-foaming agent to said container if said threshold is exceeded.

The invention will be further elucidated with reference to a figure shown in a drawing, in which figure a digester according to an embodiment of the invention is shown schematically in a vertical cross section.

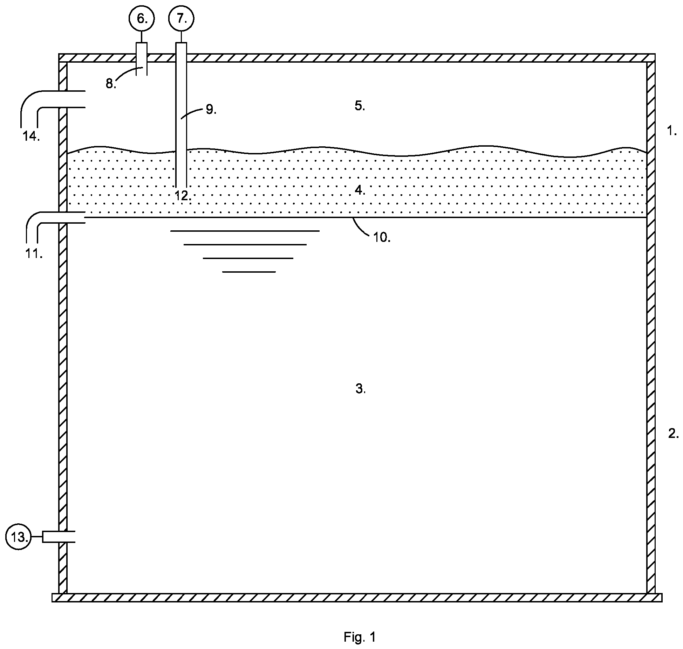

The figure shows a digester 1 with a container 2 for containing biomass and/or sludge 3 and/or a digestate thereof, up to an intended level 10. An overflow outlet 11 is provided at the level of the intended level 10, such that any excess biomass and/or sludge is discharged via said outlet 11 and said container 2 is maximally filled up to said intended level 10. Above the biomass and/or sludge 3 the container is filled with a gas/air mixture 5. Optionally, foam 4 can also be present in said container 2, in particular above the biomass and/or sludge 3. A first pressure sensor 6 is connected to a first, upper part or area of the container 2 via a relatively short tube 8. A second pressure sensor 7 is connected to a second part or area of the container 2 just above said intended level 10 via a relatively long, substantially vertically arranged tube 9. Said tube 9 extends with an opening 12 thereof just above said intended level 10. If no foam is present, both the first pressure sensor 6 and the second pressure sensor 7 measure the pressure of the gas/air mixture 5, which measured pressures are therefore substantially equal. Substantially equal pressures measured by the sensors 6, 7 is thus an indication that no foam is present in the container 2. If foam is present above the biomass and/or sludge 3, the foam will enter the tube 9 via opening 12. As a result of the foam entering the tube 9 the pressure in the tube 9 will increase, such that the second pressure sensor 7 will measure a higher pressure than the first pressure sensor 6. This pressure difference is thus an indication that foam is present in the container 2. Below the tube 8 a foam outlet 14 is provided. If any foam reaches the level of the foam outlet 14 the foam will be discharged via said outlet 14. As a result of said foam outlet 14 being arranged below the tube 8 it is easily prevented that foam enters the tube 8. Thereby the first sensor 6 will always measure the pressure of the gas/air mixture 5 and not the pressure of the foam, such that an accurate pressure difference between the gas/air mixture 5 and the foam 4 may be obtained by the first and second pressure sensors 6, 8.

As is further shown in the figure, the digester 1 in this embodiment also comprises a hydrostatic pressure sensor 13 that connects to a lower area of said container 2 and that is arranged to indicate a level of biomass and/or sludge and/or digestate thereof contained in said container 2.

It is noted that the invention is not limited to the shown embodiments but also extends to variants within the scope of the appended claims.

## Claims

1. Digester, comprising a container for containing biomass and/or sludge and/or a digestate thereof up to an intended level (10) of said container, **characterized by:**
- a first pressure sensor (6) for measuring a first pressure of gas present in said container, said first pressure sensor (6) being in medium through flow connection with a first, upper part (5) of said container, and
- a second pressure sensor (7) for measuring a second pressure of gas or foam present in said container, said second pressure sensor (7) being in medium through flow connection with a second part (4) of said container above said intended level and below said first, upper part (5), wherein
the digester comprises a tube (9) connecting said second pressure sensor (7) with said second part (4) of said container, said tube (9) having an inlet opening arranged just above said intended level.

2. Digester according to claim 1, further comprising a means arranged to compare said first and second pressures, wherein a difference in said first and second pressures is an indication of foam present in said container above said intended level (10).

3. Digester according to claim 1, wherein said first and second pressure sensors (6, 7) are integrally formed as one differential pressure sensor, said differential pressure sensor being on the one hand in said medium through flow connection with said first, upper part (5) of said container and on the other hand in said medium through flow connection with said second part (4) of said container, said differential pressure sensor being arranged to provide a difference in pressure between said first and second parts of said container as an indication of foam present in said container above said intended level (10).

4. Digester according to any of the preceding claims, said container comprising a foam outlet (14), said foam outlet (14) being arranged in an area of said container that is below said first part (5) of said container and above said second part (4) of said container.

5. Digester according to any of the preceding claims, said container comprising an overflow outlet (11), said overflow outlet (11) being arranged in an area of said container that is below said second part (4) of said container.

6. Digester according to any of the preceding claims, comprising a hydrostatic pressure sensor arranged to indicate a level of biomass and/or sludge and/or digestate thereof contained in said container.

7. Digester according to any of the preceding claims, comprising an indicating means for indicating the intended level.

8. Digester according to any of the preceding claims, wherein said second part (4) of said container is between 5 - 30 cm, preferably between 10 - 20 cm, above said intended level (10).

9. Method for detecting a presence of foam in a digester according to any of the preceding claims 1 - 8, said method comprising the steps of:
(a) filling said container with biomass and/or sludge up to said intended level (10);
(b) measuring said first and second pressures, and
(c) comparing said first and second pressures, wherein a difference in said first and second pressures is an indication of foam present in said container above said intended level (10).

10. Method according to claim 9, wherein a presence of foam in a digester according to claims 3-8 is detected, wherein step (b) and (c) are performed by measuring a difference in pressure between said first and second parts (4, 5) of said container using said differential pressure sensor.

11. Method according to claim 9 or 10, comprising the further steps of:
(d) determining if said difference obtained in step (c) exceeds a certain threshold; and
(e) providing an anti-foaming agent to said container if said threshold is exceeded.

12. Use of a digester comprising a container for containing biomass and/or sludge and/or a digestate thereof, comprising filling the digester up to an intended level (10) of said container, wherein the digester comprises:
- a first pressure sensor (6) for measuring a first pressure of gas present in said container, said first pressure sensor (6) being in medium through flow connection with a first, upper part (5) of said container, and
- a second pressure sensor (7) for measuring a second pressure of gas or foam present in said container, said second pressure sensor (7) being in medium through flow connection with a second part (4) of said container above said intended level (10) and below said first, upper part (5) wherein the use further comprises:
- measuring said first and second pressures, and
- comparing said first and second pressures, wherein a difference in said first and second pressures is an indication of foam present in said container above said intended level (10), wherein
the digester comprises a tube (9) connecting said second pressure sensor (7) with said second part (4) of said container, said tube (9) having an inlet opening arranged just above said intended level (10).

## Patentansprüche

1. Faulbehälter, der einen Behälter zum Aufnehmen von Biomasse und/oder Schlamm und/oder eines Gärrückstands davon bis zu einem geplanten Pegel (10) des Behälters aufweist, **gekennzeichnet durch**:
- einen ersten Drucksensor (6) zum Messen eines ersten Drucks von Gas, das in dem Behälter vorhanden ist, wobei der erste Drucksensor (6) in einer Mediendurchflussverbindung mit einem ersten oberen Teil (5) des Behälters ist,
- einen zweiten Drucksensor (7) zum Messen eines zweiten Drucks von Gas oder Schaum, das/der in dem Behälter vorhanden ist, wobei der zweite Drucksensor (7) in einer Mediendurchflussverbindung mit einem zweiten Teil (4) des Behälters oberhalb des geplanten Pegels und unterhalb des ersten oberen Teils (5) ist, wobei der Faulbehälter ein Rohr (9) aufweist, welches den zweiten Drucksensor (7) mit dem zweiten Teil (4) des Behälters (4) verbindet, wobei das Rohr (9) eine Einlassöffnung hat, die unmittelbar oberhalb des geplanten Pegels angeordnet ist.

2. Faulbehälter nach Anspruch 1, der ferner eine Einrichtung aufweist, die eingerichtet ist, um die ersten und zweiten Drücke zu vergleichen, wobei eine Differenz der ersten und zweiten Drücke ein Hinweis dafür ist, dass in dem Behälter Schaum über dem geplanten Pegel (10) vorhanden ist.

3. Faulbehälter nach Anspruch 1, wobei die ersten und zweiten Drucksensoren (6, 7) integral als ein Differenzdrucksensor ausgebildet sind, wobei der Differenzdrucksensor einerseits in der Mediendurchflussverbindung mit dem ersten oberen Teil (5) des Behälters und andererseits in der Mediendurchflussverbindung mit dem zweiten Teil (4) des Behälters ist, wobei der Differenzdrucksensor eingerichtet ist, um eine Druckdifferenz zwischen den ersten und zweiten Teilen des Behälters als einen Hinweis dafür, dass in dem Behälter Schaum über dem geplanten Pegel (10) vorhanden ist, bereitzustellen.

4. Faulbehälter nach einem der vorhergehenden Ansprüche, wobei der Behälter einen Schaumauslass (14) aufweist, wobei der Schaumauslass (14) in einem Bereich des Behälters eingerichtet ist, der unterhalb des ersten Teils (5) des Behälters und oberhalb des zweiten Teils (4) des Behälters ist.

5. Faulbehälter nach einem der vorhergehenden Ansprüche, wobei der Behälter einen Überlaufauslass (11) aufweist, wobei der Überlaufauslass (11) in einem Bereich des Behälters eingerichtet ist, der unterhalb des zweiten Teils (4) des Behälters ist.

6. Faulbehälter nach einem der vorhergehenden Ansprüche, der einen hydrostatischen Drucksensor aufweist, der eingerichtet ist, um einen Pegel von Biomasse und/oder Schlamm und/oder eines Gärrückstands davon, der in dem Behälter enthalten ist, anzuzeigen.

7. Faulbehälter nach einem der vorhergehenden Ansprüche, der eine Anzeigeeinrichtung zum Anzeigen des geplanten Pegels aufweist.

8. Faulbehälter nach einem der vorhergehenden Ansprüche, wobei der zweite Teil (4) des Behälters zwischen 5 und 30 cm, vorzugsweise zwischen 10 und 20 cm oberhalb des geplanten Pegels (10) ist.

9. Verfahren zum Erfassen des Vorhandenseins von Schaum in einem Faulbehälter nach einem der vorhergehenden Ansprüche 1 - 8, wobei das Verfahren die folgenden Schritte aufweist:
(a) Füllen des Behälters mit Biomasse und/oder Schlamm bis zu dem geplanten Pegel (10);
(b) Messen der ersten und zweiten Drücke, und
(c) Vergleichen der ersten und zweiten Drücke, wobei eine Differenz in den ersten und zweiten Drücken ein Hinweis dafür ist, dass in dem Behälter Schaum über dem geplanten Pegel (10) vorhanden ist.

10. Verfahren nach Anspruch 9, wobei ein Vorhandensein von Schaum in einem Faulbehälter nach den Ansprüchen 3 - 8 erfasst wird, wobei die Schritte (b) und (d) durchgeführt werden, indem eine Druckdifferenz zwischen den ersten und zweiten Teilen (4, 5) des Behälters unter Verwendung des Druckdifferenzsensors gemessen wird.

11. Verfahren nach Anspruch 9 oder 10, das ferner die folgenden Schritte aufweist:
(d) Bestimmen, ob die in Schritt (c) erhaltene Differenz einen gewissen Schwellwert überschreitet; und
(e) Bereitstellen eines Entschäumers an den Behälter, wenn der Schwellwert überschritten wird.

12. Verwendung eines Faulbehälters, der einen Behälter zum Aufnehmen von Biomasse und/oder Schlamm und/oder eines Gärrückstands davon aufweist, die das Füllen des Faulbehälters bis zu einem geplanten Pegel (10) des Behälters aufweist, wobei der Faulbehälter aufweist:
- einen ersten Drucksensor (6) zum Messen eines ersten Drucks von Gas, das in dem Behälter vorhanden ist, wobei der erste Drucksensor (6) in einer Mediendurchflussverbindung mit einem ersten oberen Teil (5) des Behälters ist, und
- einen zweiten Drucksensor (7) zum Messen eines zweiten Drucks von Gas oder Schaum, das/der in dem Behälter vorhanden ist, wobei der zweite Drucksensor (7) in einer Mediendurchflussverbindung mit einem zweiten Teil (4) des Behälters oberhalb des geplanten Pegels (10) und unterhalb des ersten oberen Teils (5) ist, wobei die Verwendung ferner aufweist:
- Messen der ersten und zweiten Drücke, und
- Vergleichen der ersten und zweiten Drücke, wobei eine Differenz in den ersten und zweiten Drücken ein Hinweis dafür ist, dass in dem Behälter Schaum über dem geplanten Pegel (10) vorhanden ist, wobei der Faulbehälter ein Rohr (9) aufweist, das den zweiten Drucksensor (7) mit dem zweiten Teil (4) des Behälters verbindet, wobei das Rohr (9) eine Einlassöffnung hat, die unmittelbar oberhalb des geplanten Pegels (10) eingerichtet ist.

## Revendications

1. Digesteur, comprenant un récipient pour contenir une biomasse et/ou une boue et/ou un digestat de celle-ci jusqu'à un niveau prévu (10) dudit récipient, **caractérisé par** :
- un premier capteur de pression (6) pour mesurer une première pression de gaz présent dans ledit récipient, ledit premier capteur de pression (6) étant en connexion d'écoulement à travers un milieu avec une première partie supérieure (5) dudit récipient, et
- un second capteur de pression (7) pour mesurer une seconde pression de gaz ou de mousse présent dans ledit récipient, ledit second capteur de pression (7) étant en connexion d'écoulement à travers un milieu avec une seconde partie (4) dudit récipient au-dessus dudit niveau prévu et en dessous de ladite première partie supérieure (5), dans lequel le digesteur comprend un tuyau (9) reliant ledit second capteur de pression (7) à ladite seconde partie (4) dudit récipient, ledit tuyau (9) ayant une ouverture d'entrée agencée juste au-dessus dudit niveau prévu.

2. Digesteur selon la revendication 1, comprenant en outre un moyen agencé de façon à comparer lesdites première et seconde pressions, dans lequel une différence entre lesdites première et seconde pressions est une indication de mousse présente dans ledit récipient au-dessus dudit niveau prévu (10).

3. Digesteur selon la revendication 1, dans lequel lesdits premier et second capteurs de pression (6, 7) sont intégralement formés sous forme d'un capteur de pression différentielle, ledit capteur de pression différentielle étant d'une part en connexion d'écoulement à travers un milieu avec ladite première partie supérieure (5) dudit récipient et d'autre part en connexion d'écoulement à travers un milieu avec ladite seconde partie (4) dudit récipient, ledit capteur de pression différentielle étant agencé de façon à fournir une différence de pression entre lesdites première et seconde parties dudit récipient en tant qu'indication de mousse présente dans ledit récipient au-dessus dudit niveau prévu (10).

4. Digesteur selon l'une quelconque des revendications précédentes, ledit récipient comprenant une sortie de mousse (14), ladite sortie de mousse (14) étant agencée dans une zone dudit récipient qui est en dessous de ladite première partie (5) dudit récipient et au-dessus de ladite seconde partie (4) dudit récipient.

5. Digesteur selon l'une quelconque des revendications précédentes, ledit récipient comprenant une sortie de débordement (11), ladite sortie de débordement (11) étant agencée dans une zone dudit récipient qui est en dessous de ladite seconde partie (4) dudit récipient.

6. Digesteur selon l'une quelconque des revendications précédentes, comprenant un capteur de pression hydrostatique agencé de façon à indiquer un niveau de biomasse et/ou de boue et/ou de digestat de celle-ci contenus dans ledit récipient.

7. Digesteur selon l'une quelconque des revendications précédentes, comprenant un moyen d'indication pour indiquer le niveau prévu.

8. Digesteur selon l'une quelconque des revendications précédentes, dans lequel ladite seconde partie (4) dudit récipient est entre 5 et 30 cm, de préférence entre 10 et 20 cm, au-dessus dudit niveau prévu (10).

9. Procédé de détection d'une présence de mousse dans un digesteur selon l'une quelconque des revendications précédentes 1 à 8, ledit procédé comprenant les étapes de :
(a) remplissage dudit récipient avec une biomasse et/ou une boue jusqu'audit niveau prévu (10) ;
(b) mesure desdites première et seconde pressions, et
(c) comparaison desdites première et seconde pressions, dans lequel une différence desdites première et seconde pressions est une indication de mousse présente dans ledit récipient au-dessus dudit niveau prévu (10).

10. Procédé selon la revendication 9, dans lequel une présence de mousse dans un digesteur selon les revendications 3 à 8 est détectée, dans lequel les étapes (b) et (c) sont réalisées par mesure d'une différence de pression entre lesdites première et seconde parties (4, 5) dudit récipient à l'aide dudit capteur de pression différentielle.

11. Procédé selon la revendication 9 ou 10, comprenant les étapes supplémentaires de :
(d) détermination du fait que ladite différence obtenue dans l'étape (c) dépasse un certain seuil ; et
(e) fourniture d'un agent anti-mousse dans ledit récipient si ledit seuil est dépassé.

12. Utilisation d'un digesteur comprenant un récipient pour contenir une biomasse et/ou une boue et/ou un digestat de celle-ci, comprenant le remplissage du digesteur jusqu'à un niveau prévu (10) dudit récipient, dans lequel le digesteur comprend :
- un premier capteur de pression (6) pour mesurer une première pression de gaz présent dans ledit récipient, ledit premier capteur de pression (6) étant en connexion d'écoulement à travers un milieu avec une première partie supérieure (5) dudit récipient, et
- un second capteur de pression (7) pour mesurer une seconde pression de gaz ou de mousse présent dans ledit récipient, ledit second capteur de pression (7) étant en connexion d'écoulement à travers un milieu avec une seconde partie (4) dudit récipient au-dessus dudit niveau prévu (10) et en dessous de ladite première partie supérieure (5), dans laquelle l'utilisation comprend en outre :
- la mesure desdites première et seconde pressions, et
- la comparaison desdites première et seconde pressions, dans laquelle une différence desdites première et seconde pressions est une indication de mousse présente dans ledit récipient au-dessus dudit niveau prévu (10), dans laquelle le digesteur comprend un tuyau (9) reliant ledit second capteur de pression (7) avec ladite seconde partie (4) dudit récipient, ledit tuyau (9) ayant une ouverture d'entrée agencée juste au-dessus dudit niveau prévu (10).
